# EUROPEAN PATENT APPLICATION

(11) **EP 4 176 828 A1**
(43) Date of publication of application: **10.05.2023**
(21) Application number: 22205014.8
(22) Date of filing: 02.11.2022
(51) Int. Cl.: A61B 17/221

(54) **CATHETER WITH LOW-SHEAR TIP**

(30) Priority: 03.11.2021 US 202117518401
(71) Applicant: Neuravi Limited, Galway H91 K5YD (IE)
(72) Inventor: VALE, David, Galway, H91 K5YD (IE); KELLY, Ronald, Galway, H91 K5YD (IE); CASEY, Brendan, Galway, H91 K5YD (IE); KEATING, Karl, Galway, H91 K5YD (IE)
(74) Representative: Carpmaels & Ransford LLP

(57) **Abstract**

The designs and methods disclosed herein are for a clot retrieval catheter with a large bore shaft and a distal braid-supported tip that is expandable. A distal expansile section of the braid-supported tip can have a delivery inner diameter approximately equal to the inner diameter of the shaft. The braid of the tip can have a decreasing braid angle distally so that the tip can expand to a larger expanded inner diameter when impinged radially by an ingested clot to manage the clot and prevent shearing of clot fragments. The wires of the tip braids can follow one spiral direction distally and then invert proximally back on themselves to form the other spiral direction of the braid. This inversion of the wires results in atraumatic distal hoops at the distal termination of the braid. Designs can further have spines capable of resisting elongation of the catheter shaft during a procedure.

## Description

### Field of Invention

The present invention generally relates devices and methods for removing acute blockages from blood vessels during intravascular medical treatments. More specifically, the present invention relates to retrieval catheters with expandable tips into which an object or objects can be retrieved.

### Background

Clot retrieval aspiration catheters and devices are used in mechanical thrombectomy for endovascular intervention, often in cases where patients are suffering from conditions such as acute ischemic stroke (AIS), myocardial infarction (MI), and pulmonary embolism (PE). Accessing the neurovascular bed in particular is challenging with conventional technology, as the target vessels are small in diameter, remote relative to the site of insertion, and highly tortuous. These catheters are frequently of great length and must follow the configuration of the blood vessels in respect of all branching and windings. Traditional devices are often either too large in profile, lack the deliverability and flexibility needed to navigate particularly tortuous vessels, or are not effective at removing a clot when delivered to the target site.

Many existing designs for aspiration retrieval catheters are often restricted to, for example, inner diameters of 6Fr or between approximately 0.068 - 0.074 inches. Larger sizes require a larger guide or sheath to be used, which then necessitates a larger femoral access hole to close. Most physicians would prefer to use an 8Fr guide / 6Fr sheath combination, and few would be comfortable going beyond a 9Fr guide / 7Fr sheath combination. This means that once at the target site, a clot can often be larger in size than the inner diameter of the aspiration catheter and must otherwise be immediately compressed to enter the catheter mouth. This compression can lead to bunching up and subsequent shearing of the clot during retrieval. Firm, fibrin-rich clots can also become lodged in the fixed-mouth tip of these catheters making them more difficult to extract. This lodging can also result in shearing where softer portions breaking away from firmer regions of the clot.

Large bore intermediate and aspiration catheters and/or those with expandable tips are therefore desirable because they provide a large lumen and distal mouth to accept a clot with less resistance. The bore lumen of these catheters can be nearly as large as the guide and/or sheath through which they are delivered, and the expandable tip can expand to be larger still. When a clot is captured and drawn proximally into an expandable tip, the clot can be progressively compressed and sheltered during retrieval so that it can be aspirated fully through the catheter and into a syringe or cannister.

In many examples, the fixed-mouth catheters and those with expandable tips can have an underlying braid as the primary supporting backbone. The use of braids in a catheter body is not a novel concept, and typical examples can be readily found in the art. The braid can often be as simple as bands wrapped spirally in one direction for the length of the catheter which cross over and under bands spiraled in the opposite direction. The bands can be metallic, fiberglass, or other material providing effective hoop strength to reinforce the softer outer materials of the body. However, supporting braids can often lack an effective bonding mechanism for the layers, or have a high sectional stiffness the point where they do not meet the flexibility criteria for many procedures. Additionally, many of these devices have structures or profiles which cannot be made soft enough for use in fragile vessels without causing substantial trauma. Furthermore, many catheter braids lack the capacity for radial expansion, leading to a clot bunching up and lodging in the mouth of the tip, blocking the lumen. This necessitates removal of the entire catheter from the patient and sanitizing it and any other devices prior to making subsequent passes, increasing the time and risk involved.

Combining the clinical needs of these catheters without significant tradeoffs can be tricky. Catheter designs attempting to overcome the above-mentioned design challenges would need to have a large bore but must also have a small enough outer diameter to reach distal targets with the flexibility and elasticity to survive the severe mechanical strains imparted when navigating the tortuous vasculature. The designs can have an expandable tip capable of elastic radial expansion as a clot is ingested for better retention of the clot during this critical phase., while capable of quickly recovering its initial shape to maintain the outer diameter to facilitate the passage of the tip to more distal vessels and through devices while maintaining the passageway for aspiration.

As a result, there remains a need for improved catheter designs attempting to overcome the above-mentioned design challenges. The present designs are aimed at providing an improved retrieval catheter with an expansile tip section and methods for using such a catheter capable improved performance.

### Summary

It is an object of the present designs to provide devices and methods to meet the above-stated needs. The designs can be for a clot retrieval catheter capable of remove a clot from cerebral arteries in patients suffering AIS, from coronary native or graft vessels in patients suffering from MI, and from pulmonary arteries in patients suffering from PE and from other peripheral arterial and venous vessels in which a clot is causing an occlusion. The designs can also resolve the challenges of aspirating fibrin rich clot material by addressing the key difficulties of 1) the friction between the clot and the catheter and 2) the energy/work required to deform these firm clots as they are aspirated into the catheter tip.

In some examples, a catheter can be a large bore catheter with a low shear tip having a proximal elongate shaft with a proximal end, a distal end, an internal lumen, and a longitudinal axis extending therethrough. The elongate shaft can have a low friction inner liner and a first plurality of wire braided segments disposed around the liner. The braided segments can serve as the backbone and support for the catheter shaft. The interlacing weave of the braid can form circumferential rings of cells around the axis of the elongate shaft.

In some examples, the catheter can have a distal tip section having the same nominal inner diameter as the elongate shaft extending from the distal end of the elongate shaft. The tip section can be divided into several regions; a proximal tubular body, and a distal expansile section having a delivery configuration and an expanded clot capture configuration. The distal expansile section of the tip can have a larger expanded inner diameter when impinged radially by an ingested clot in the expanded clot capture configuration and a smaller delivery inner diameter in the delivery configuration. These capabilities manage the clot and prevent shearing of clot fragments.

This innovation of using the clot to expand the low shear tip section as needed can allow for much improved clot management over traditional designs. The nominal, non-expanded outer diameter maximizes distal access to a target area. Once a clot is ingested, accommodating fibrin rich portions of the clot through additional radial expansion can compress the clot more progressively and lead to significantly less clot shearing than catheters that lack this capability.

The support for the tip section can be a second plurality of wire braided segments, with the overlapping wires forming circumferential rings of cells. In some examples, the second plurality of wire braided segments can be formed monolithically with the first plurality of wire braided segments of the shaft. The wires of the second plurality of wire braided segments can follow one spiral direction distally from the proximal end of the tip section, and then invert proximally back on themselves at the distal end of the tip section to form the other spiral direction of the braid. This inversion of the wires results in atraumatic distal hoops at the distal termination of the tip braided segments.

The catheter can also have one or more axial spine members extending along the longitudinal axis from the proximal end of the first plurality of wire braided segments of the elongate shaft. Spines can resist elongation of the catheter shaft tensile loads during a procedure. The spine can run along the inner surface of the braids, along the outer surface of the braids, or both. In one example, the spine is interwoven through the cells of the braids. In another example, the spine can have a spiral or helical pattern around the axis.

In some examples, the spine can invert proximally at a loop point through an opening in a cell of the second plurality of wire braided segments. The spine loop point can be located at a distance proximal of the distal end of the tip section. In one example, this location is approximate the distal end of the inner liner. In another example, the distance can be specified as approximately 4-5 mm proximal of the distal end of the tip section. After inverting at the loop point, the spine can extend proximally for a fixed longitudinal distance and be secured or extend all the way to the proximal end of the elongate shaft.

In some examples, the spine or spines can be stiff, solid members of polymeric or metallic materials or can be of compound construction using a core and multiple materials. Other examples, the spine can be a thread or other structure capable of supporting tensile loads but not compressive loads. In one example, the spine can be a polymeric thread such as a liquid crystal polymer (LCP) which resists tensile elongation but allowing compressive shortening. This spine thread structure can perform its tensile role while contributing very little to the lateral flexibility of the catheter shaft.

In another example, the tensile strength of the assembly can be increased by using novel liners that maintain lateral flexibility and frictional properties of the lumen while offering greatly increased tensile strength. Such liners are readily commercially available from Junkosha and consist of a wrapped ePTFE structure.

The catheter can have one or more radiopaque marker bands to identify various transition points and terminal ends of the device during a procedure. The marker bands can be platinum, gold, and/or another metallic collar, or alternatively can be coated with a compound giving substantial radiopacity. For example, a distal band can be crimped onto the catheter shaft a distance approximately 10 mm from the distal end of the expandable tip. The axial length between the distal end of the inner liner and the distal end of the tip section can also be between approximately 1 mm and approximately 10 mm. In a more specific example, the length between the distal end of the inner liner and the distal end of the tip section can be between approximately 2 mm and approximately 3 mm.

In some examples, the first and second plurality of wire braided segments can be formed as a single monolithic structure. Alternately, one or more of the marker bands can also be used as structural joints within the catheter shaft. In one example, a proximal marker band at an intermediate length of the catheter shaft can overlap axially with the distal end of the first plurality of wire braided segments and the proximal end of the second plurality of wire braided segments. The bond of the joint can then be formed though welding, adhesives, or other suitable mechanical linkage. If the catheter length is the typical 1250 mm to 1320 mm of some designs, the second plurality of wire braided segments can have an overall longitudinal length in the range of approximately 100 to approximately 400 mm, thereby positioning the joint with the proximal marker band at an approximate distance within this range from the distal end of the catheter. The overall longitudinal length of the tip section can thus be from approximately 100 up to approximately 400 mm.

The cells of the first plurality of wire braided segments and the second plurality of wire braided segments can be braided in particular patterns to give differing mechanical properties to different portions of the catheter. For example, the angle formed by wire crossover in the cells, and the density in programmable pics per inch (PPI) can be tailored for a higher hoop strength catheter shaft proximally. The angles and PPI can transition to an arrangement in the distal tip that has a lower hoop strength to promote deliverability and allow the capacity for additional radial expansion of the tip as a clot is ingested. Moreover, gradual transitions can be made between differing PPI and cell angles to avoid the formation of unwanted kink points of stress concentrations.

In some examples, each cell of the first plurality of wire braided segments in the proximal elongate shaft can have a first braided segment braid angle. The first plurality of wire braided segments can also have a relatively dense picks per inch in a range of approximately 120 to approximately 170. A denser braid with a large cell angle can give good pushability, kink resistance, and bending stiffness. Alternately, a lower, more flexible PPI of 50-80 can be utilized with a reinforcing wire coil to further improve kink resistance at a lower bending stiffness.

At least a portion of the second plurality of wire braided segments of the expandable low shear distal tip section are capable of radial expansion, and therefore can have variable PPI and cell angles to balance allowable expansion with radial force capabilities. The second plurality of wire braided segments can have at least a first proximal braid angle and a final distal braid angle smaller than the first braid angle. In some examples, the first and final braid angles can have a range between approximately 65 degrees to approximately 160 degrees.

In one design, these braids can have an initial proximal PPI of approximately 140 and an initial proximal braid angle of approximately 154 degrees. The final braid angle of the distalmost cells of the second plurality of wire braided segments can have a range between approximately 40 degrees to approximately 125 degrees. In another example, the final braid angle of the distalmost cells can have a range between approximately 85 degrees to approximately 95 degrees.

In a more specific example, the distal rings of cells containing the distalmost cells of the second plurality of wire braided segments in the tip can have a PPI in a range of approximately 20 - 45. In another specific example, the initial proximal PPI and braid angle can transition to a final distal PPI and final braid angle of approximately 21 and 65 degrees, respectively. In another example, the final PPI and final braid angle can be approximately 36 and 95 degrees, respectively. Other balances of final braid density and angle can similarly be contemplated.

Other properties of wire braided segments can also be tailored for certain properties. In some examples, portions of the first plurality of wire braided segments can have a wire diameter different than at least a portion of the wire diameter in the second plurality of braids. In one instance, the first plurality of braids can have wires having a thickness of approximately 0.0015 inches or some other diameter. The second plurality of braids can have wires with a thickness of approximately 0.0020 inches or some other diameter.

The wires can also assume a non-circular cross sectional shape or have custom or irregular braid patterns to affect localized properties of the catheter. In one example, the first plurality of braids can have at least one section with a 1 wire under 2 over 2 herringbone pattern and be laser welded at the distal end to the proximal marker band. The second plurality of wire braided segments can have a 1 over 1 half-diamond pattern in at least a portion of the distal tip section and be welded at the proximal end to the proximal marker band.

In some examples, at least a portion of the first plurality of wire braided segments can have wires with a first material composition different than a second material composition in at least a portion of the second plurality of wire braided segments. In one case, the proximal first plurality of wire braided segments can be a stainless steel composition. In another example, the distal wires of the second plurality of wire braided segments can be of Nitinol or another superelastic alloy composition allowing for greater flexibility and also improving resistance to plastic deformation.

The supporting braid structure of the elongate shaft and expandable low shear tip can be covered with a plurality of outer polymeric jackets. In some examples, one or more polymer body jackets can be disposed around the elongate shaft and one or more polymer tip jackets can be disposed around the tip section. The tip and shaft outer jackets can be formed together or separately using polymer reflow, injection molding, or other suitable processes.

These outer jackets can have varying durometer hardness to create a proximal portion with more column stiffness and transition into a distal portion with more lateral flexibility. In some examples, the body jackets can have a hardness in the range between approximately 25 to approximately 72 Shore D. The tip jackets can generally have lesser hardness with a distalmost tip jacket having the softest jacket for the most atraumatic vessel crossing profile. In one example, the distalmost tip jacket can have a hardness in the range between approximately 40 Shore A to approximately 80 Shore A.

Different jacket thicknesses can also be used. In one example, at least a portion of the plurality of body jackets can have a first wall thickness less than the wall thickness of at least a portion of the plurality of tip jackets. In one example, the body jackets can have a wall thickness of 0.003 to 0.004 inches, ideally with greater than 0.0005 inches of jacket wall thickness residing above the braid wires. In another example, the tip jackets can have a greater wall thickness of 0.006 inches. The increased thickness can aid in compressing the clot and resisting the forces of aspiration.

The distalmost tip jacket can be trimmed to follow the desired contours of the tip or can extend a longitudinal distance distally to overhang beyond the distal end of the hoops of the second plurality of wire braided segments. In some examples, the longitudinal distance of the overhang can be in a range from approximately 0.1 mm to approximately 1.0 mm. In a more specific example, this longitudinal distance can be in a range between 0.4 mm to approximately 0.6 mm.

A method for treating an occlusive clot in a target blood vessel using a catheter of the designs herein can also be disclosed. The method can include advancing a catheter system comprising an innermost aspiration catheter and a plurality of outer aspiration catheters towards the target clot. The innermost catheter in the system can have a wire braided structure with a nominal inner diameter and configured to radially expand to a larger expanded inner diameter due to the passive forces from ingesting the clot or pulling a stentriever through it. The innermost catheter can safely expand to seal or leave a small gap with the target vessel. It can also be desirable that each catheter in the system have this capability to further expand for better force influence on the captured clot and lessen the risk of shearing portions of a composite clot.

At least one of the outer aspiration catheters in the system can be a funnel super-bore catheter with a large inner lumen and a radially self-expanding distal tip for improved aspiration efficiency and better protection when retrieving the captured clot. The method can then involve maintaining the position of each of the plurality of outer aspiration catheter when the outer diameter of each successive outermost aspiration catheter matches the inner diameter of the vessel being traversed. This telescoping system can help ensure there is minimal step between catheters and that the catheter with the largest possible outer diameter is used to ingest the clot for enhanced aspiration suction and diminished risk of clot shearing. When the funnel catheter is deployed from the preceding outer catheter to radially expand the self-expanding tip, it can be advanced with the tip in the expanded configuration until the outer diameter of the tip matches the inner diameter of the vessel being traversed or when the tip has been advanced to its lower vessel size range in the case where the tip conformability allows advancement into vessels that are smaller than its fully expanded size. In many cases, the tip can seal with the vessel to prevent the flow of fluid from locations proximal of the tip.

In some examples, the method can then include advancing distally the innermost aspiration catheter, which can be sized for the nominal outer diameter as the inner diameter of the target vessel, telescopically through the catheter system to a location just proximal of the clot. Aspiration can be directed through the at least the innermost aspiration catheter to ingest the clot. Aspiration can be directed through other catheters in the system as well. For obdurate clots, an additional step in the method can then involve advancing a clot retrieval device through the lumen of the innermost aspiration catheter or through a microcatheter that is advanced through the lumen of the innermost catheter to aid in capturing the clot.

The method can entail utilizing local radial forces generated as the clot is ingested proximally through the tip section of the innermost aspiration catheter to radially expand the wire braided structure to an outer diameter larger than the nominal outer diameter of the structure. This localized effect further compresses the clot while maintaining the low profile of other sections.

In some examples, the expansion of the limited section of the catheter tip caused by the clot can create a plunger-type effect between the outer wall of the innermost aspiration catheter and the inner wall of the preceding catheter in the system when the catheter is retracted through an outer catheter. The method can then involve using this effect to generate a suction by proximally retracting the innermost aspiration catheter with the radially expanded expandable section and clot through the lumen of the next outer catheter/funnel catheter to aspirate any remaining clot fragments. Aspiration can continue to be directed through one or more of the plurality of outer aspiration catheters to assist with this process.

Wherever possible, at least the outermost catheter in the system can be left in place while the clot is withdrawn to maintain access to the target site. Contrast can be injected to determine the extent to which the vessel patency is determined, and additional passes can be made with the inner catheter(s) as desired.

Other aspects of the present disclosure will become apparent upon reviewing the following detailed description in conjunction with the accompanying figures. Additional features or manufacturing and use steps can be included as would be appreciated and understood by a person of ordinary skill in the art.

### Brief Description of the Drawings

The above and further aspects of this invention are further discussed with reference to the following description in conjunction with the accompanying drawings, in which like numerals indicate like structural elements and features in various figures. The drawings are not necessarily to scale, emphasis instead being placed upon illustrating principles of the invention. The figures depict one or more implementations of the inventive devices, by way of example only, not by way of limitation. It is expected that those of skill in the art can conceive of and combine elements from multiple figures to better suit the needs of the user.
FIG. 1 is views of an aspirating clot retrieval catheter with an expandable tip being advanced through the vasculature according to aspects of the present invention;
FIG. 2 is a segmented view of an aspirating clot retrieval catheter with an expandable tip according to aspects of the present invention;
FIG. 3 illustrates the distal portion of the aspirating clot retrieval catheter of FIG. 2 according to aspects of the present invention;
FIG. 4A is a view of an aspirating clot retrieval catheter with an expandable tip just prior to ingesting a clot according to aspects of the present invention;
FIG. 4B demonstrates the aspirating clot retrieval catheter with the expandable tip just after ingesting a clot according to aspects of the present invention;
FIG. 5 shows a cutaway view of an aspirating clot retrieval catheter according to aspects of the present invention;
FIG. 6 depicts the proximal end of the large bore clot retrieval catheter of FIG. 5 according to aspects of the present invention;
FIG. 7 shows the distal end of the large bore clot retrieval catheter of FIG. 5 according to aspects of the present invention;
FIG. 8A illustrates an example braid configuration for the distal tip section according to aspects of the present invention;
FIG. 8B shows another example braid configuration for the distal tip section according to aspects of the present invention;
FIGs. 9A-9H demonstrate steps of a method for the construction of a catheter according to aspects of the present invention;
FIGs. 10A-10B are cross section views of the braid inversion at the distal end of the catheter tip according to aspects of the present invention;
FIG. 11 illustrates a telescoping method utilizing multiple aspirating clot retrieval catheter with expandable tips according to aspects of the present invention;
FIG. 12 depicts the telescoping catheters of FIG. 11 in use in the vasculature according to aspects of the present invention;
FIG. 13 shows a telescoping method utilizing an aspirating clot retrieval catheter with an expandable in conjunction with a funnel catheter with a self-expanding tip according to aspects of the present invention; and
FIG. 14 is a flow diagram of a method of use for an aspirating clot retrieval catheter according to aspects of the present invention.

### Detailed Description

Specific examples of the present invention are now described in detail with reference to the Figures, where identical reference numbers indicate elements which are functionally similar or identical. The examples address many of the deficiencies associated with traditional clot retrieval aspiration catheters, such as poor or inaccurate deployment to a target site and ineffective clot removal.

The designs herein can be for a clot retrieval catheter with a large bore lumen and a distal low shear tip that can expand to a diameter larger the nominal diameter when it interacts with an ingested clot or stentriever. The designs can have a proximal elongate body for the shaft of the catheter, and a distal tip with an expanding braided support structure and outer polymeric jacket to give the tip atraumatic properties. Portions of the tip section can be designed with the ability to further expand beyond the nominal diameter when ingesting a clot using braid cells capable of easily and repeatably collapsing for delivery and expanding locally under loading from the clot. This management of the clot during ingestion can significantly reduce shearing of the clot. The catheter's design can be sufficiently flexible to navigate highly tortuous areas of the anatomy and be able to recover its shape to maintain the inner diameter of the lumen when displaced in a vessel.

This innovation of utilizing the clot itself to expand the tip section as needed allows for much improved clot handling and less shearing over traditional designs. The nominal, non-expanded outer diameter maximizes distal access reach like a standard fixed-mouth catheter. Once a clot is subsequently ingested, accommodating stiff, fibrin-rich portions of the clot through additional radial expansion can gradually compress the clot such that there is significantly less clot shearing than catheters that lack this capability. Further, the conformable nature of the tip allows it to be advanced atraumatically past calcified lesions without dislodging plaque material.

Accessing the various vessels within the vascular, whether they are coronary, pulmonary, or cerebral, involves well-known procedural steps and the use of a number of conventional, commercially-available accessory products. These products, such as angiographic materials, mechanical thrombectomy devices, microcatheters, and guidewires are widely used in laboratory and medical procedures. When these products are employed in conjunction with the devices and methods of this invention in the description below, their function and exact constitution are not described in detail. Additionally, while the description is in many cases in the context of thrombectomy treatments in intercranial arteries, the disclosure may be adapted for other procedures and in other body passageways as well.

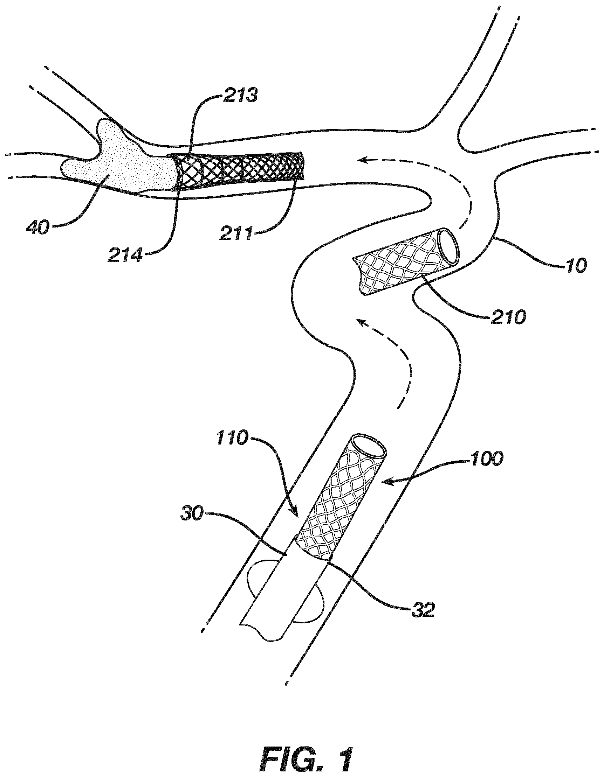

Turning to the figures, FIG. 1 illustrates a possible sequence for approaching an occlusive clot 40 using a large bore aspirating clot retrieval catheter 100 of the designs disclosed herein. The clot 40 can be approached with the catheter 100 collapsed within a guide sheath 30 or another outer catheter. When the vasculature 10 becomes too narrow and/or tortuous for further distal navigation with the guide sheath 30, the catheter 100 can be deployed for further independent travel distally. The catheter 100 can be highly flexible such that it is capable of navigating the M1 or other tortuous regions of the neurovascular to reach an occlusive clot.

The clot retrieval catheter 100 can have a flexible elongate body 110 serving as a shaft with a large internal bore (which in some cases can be 0.090 inches or larger) and a distal tip section 210 having a supporting braided structure. The large bore helps the catheter to be delivered to a target site by a variety of methods. These can include over a guidewire, over a microcatheter, with a dilator/access tool, or by itself.

In many cases, the design of the shaft and tip can be configured so that the catheter 100 can be delivered through (and retrieved back through) common outer sheaths and guides. For example, a standard 6Fr sheath / 8Fr guide, would typically have an inner lumen of less than 0.090 inches. The catheter can then be designed with a nominal outer diameter of approximately 0.086 inches. Although the supporting braid design can be different than that of the shaft, the tip section 210 must be designed to be able to resist collapse from the forces of aspiration, have excellent lateral flexibility in both the expanded and collapsed states, and an atraumatic profile to prevent snagging on bifurcations in vessels.

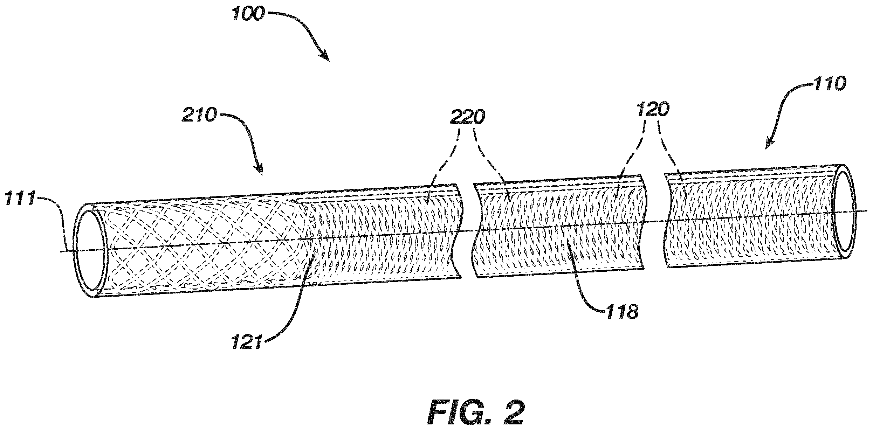

A view showing important sections of a catheter 100 of this disclosure is illustrated in FIG. 2. The elongate shaft 110 can have a backbone consisting of a first plurality of braid sections 120 enclosed by an axial series of outer body jackets. As used herein, "braided sections" can refer to segments within a single monolithic braid that have different physical properties and/or configurations and does not necessarily mean two distinct structures bonded together.

Similarly, the tip section 210 can have another series of braided sections 220 surrounded by one or more polymeric tip jackets. In some designs the braided sections 220 of the tup section 210 can be formed monolithically with the braid sections 120 of the shaft. It can be appreciated that different braided sections of the tip and shaft braids can have different geometries and weave patterns to achieve desired properties for that segment of catheter.

Radiopaque marker bands can be included at different axial points along the length of the catheter 100 for visibility under fluoroscopy during a procedure. In the example illustrated, a proximal marker band 118 can illuminate the joint between the shaft 110 and the tip section 210 to give an attending physician an indication of where the more flexible and expansile capability of the catheter begins. The band 118 shown can be platinum strip or other noble metal with a relatively short length of between approximately 0.025 - 0.030 inches and a thin wall thickness (approximately 0.0005 inches) to minimize the impact on flexibility and the outer diameter of the catheter. Similarly, a more distal marker band 121 can be situated downstream to mark the terminal end of the catheter in use.

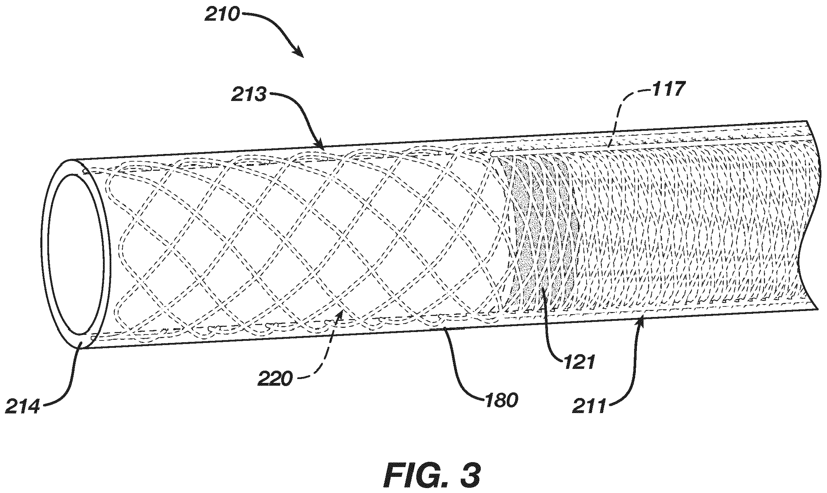

A closer view of the distal portion of the catheter with the tip section 210 is illustrated in FIG. 3. The tip section 210 can have a first substantially tubular proximal portion 211 and a second distal expansile section 213. The distal expansile section 213 can be nominally tubular, but changes to the braid properties can be more pronounced to allow for radial expansion when a large, stiff clot is swallowed into the distal mouth of the tip. This expansion can reduce shearing and improve the chances of first pass success.

The outer tip jackets 180 can be formed from a highly-elastic material such that the radial force exerted by expanding the expansile section 213 is sufficient to stretch the jackets to the enlarged state. One example can be using a ductile elastomer which has the advantages of being soft and flexible with resistance to tearing and perforation due to a high failure strain. Alternately, the jacket can be baggy and loose and fold over the support frame edges so that the braid can move more freely when expanded and collapsed.

One or more axial spines 117 can be used as an additional link between the shaft body and the tip section 210. The spine or spines 117 can counteract tensile elongation and contribute to the push characteristics of the shaft. This can be especially beneficial for the expandable tip as a large stiff clot can become lodged at the distal end of the catheter and can subject it to large tensile forces as the catheter is retracted into a larger outer sheath for removal from the vessel. The spine can be positioned beneath the braid, threaded between weaves of the braid, located on the outer diameter of the braid, or some combination of these. The spine can be composed of metallics, a polymeric, or composite strands such as Kevlar. In some preferred examples a liquid crystal polymer (LCP), such as Technora, can be utilized which is easy to process and offers high tensile strength without sacrificing any lateral flexibility.

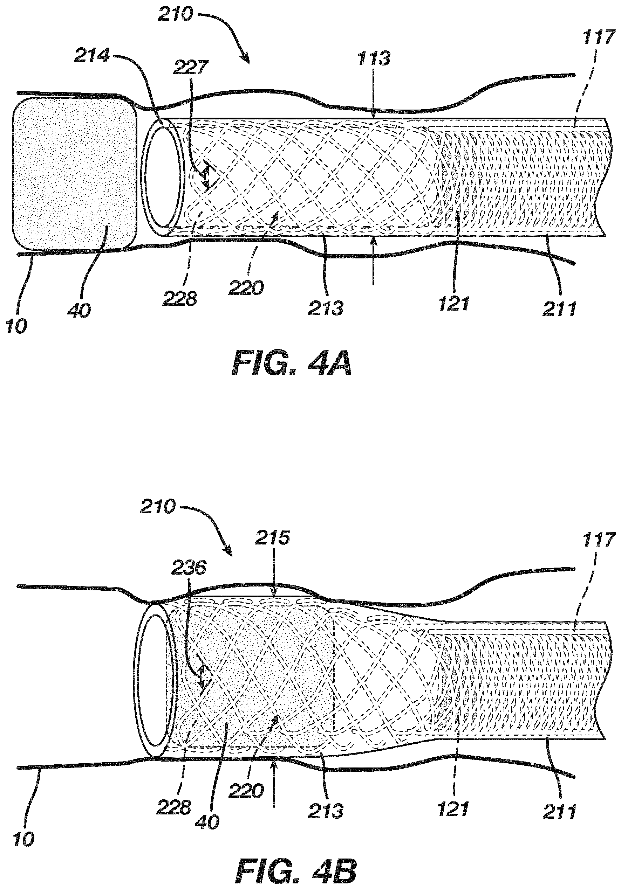

A possible sequence for clot interaction with the catheter is illustrated in FIG. 4A and FIG. 4B. The elongate shaft 110 and low shear tip section 210 can be sized to be compatible with relatively low-profile access sheaths and catheters, so that a puncture wound in the patient's groin (in the case of femoral access) can be easily and reliably closed. For example, the catheter 100 can be required to pass through the lumen of a sheath or guide with an inner diameter of less than 0.110 inches, preferably 0.090 inches, in some cases less than 0.087 inches, and most preferably less than 0.085 inches. Therefore, a standard 6Fr catheter shaft and tip section can have an overall delivery profile with a nominal, unexpanded inner diameter 113 of approximately 0.070 - 0.072 inches (0.084 inches or 2 mm outer diameter) for as much distal access and reach as possible for remote targets.

In FIG. 4A, the catheter can be deployed from an outer guide or sheath (not shown) and advanced independently to the location of a clot 40 in the vasculature 10. The plurality of braids 220 forming the backbone of the tip section 210 can have a less dense PPI weave than more proximal regions and more acute braid angles 227 in the cells 228 leading up to the distal end 214, giving the expansile section 213 the capability to enlarge. For example, a distal braid angle 227 can be approximately 60 degrees and increase to 110 degrees or more at a distance of between 5-10 mm from the distal end 214. This is particularly beneficial for clots having large fibrin-rich portions which cannot be easily compressed and may otherwise shear away or lodge in the tip of more traditional fixed-mouth catheters. Alternately, or in addition to, the tip section can also be expanded by a clot retrieval device such as a stentriever with sufficient radial force.

As a clot 40 is ingested into the tip under aspiration, it can impinge on and expand the tip locally to protect and shelter the clot, as seen in FIG. 4B. The tip section assumes an expanded clot capture configuration as the distal cell angles 236 expand so the distal expansile section 213 assumes an expanded inner diameter 215 locally around the clot that is a function of how much of the clot was able to be compressed. This is important because a clot must be deformed in order to fit into the catheter lumen, and firm clots with high coefficients of friction do not tend to deform and reshape easily and thus do not readily conform to the passive shape of the catheter tip. Reactive expansion of the tip section can allow the catheter tip to affect a seal and consequently increase a suction grip on the clot so that it can be retracted to safety. Even if the clot cannot be fully deformed into the lumen of the catheter, internalizing it within the tip section can better secure it for removal, and prevent shearing and the potential for snagging on bifurcations or other parts of the anatomy.

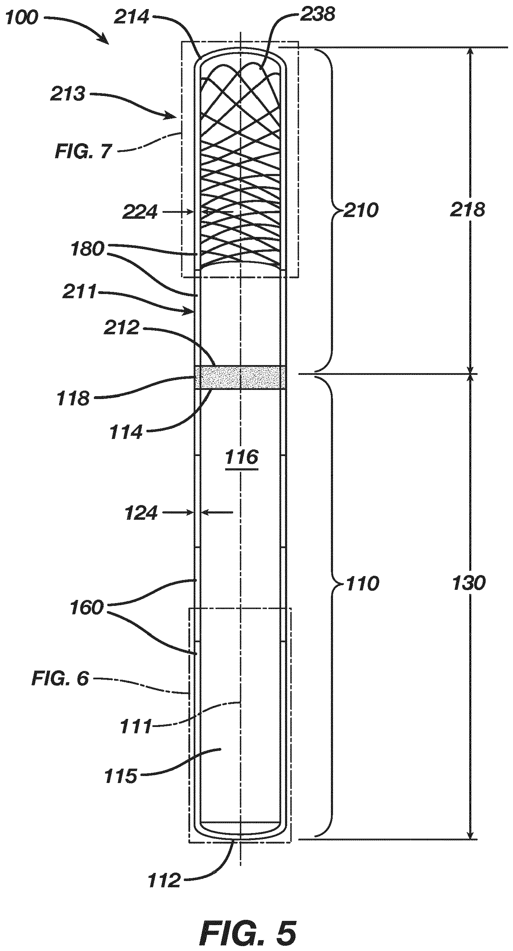

FIG. 5 shows a cutaway view of a design for an aspirating clot retrieval catheter 100. The catheter 100 can have a proximal tubular elongate shaft 110 configured around a longitudinal axis 111. The elongate shaft 110 can have an underlying braided support structure (not shown for clarity) defining the catheter lumen 116, which can have a low friction inner liner 115 and be used for the delivery of auxiliary devices, contrast injection, and direct distal aspiration to a clot face. Preferred liner materials can be polytetrafluoroethylene (PTFE), wrapped ePTFE, or similar materials. A distal tip section 210 can extend distal to the shaft and loaded within an outer sheath for delivery. The shaft 110 and tip section 210 can be concentric with the longitudinal axis 111 and share the same approximate diameter for smooth delivery through the outer sheath.

The outer surface of the elongate shaft 110 can be a series of five or six outer polymeric body jackets 160. The jackets can be made of various medical grade polymers, such as PTFE, polyether block amide (Pebax^{®}), or Nylon. Materials can be chosen, for example, so that progressively more proximal segments are generally harder and less flexible (by durometer hardness, flexure modulus, etc.) for pushability as the proximal end 112 of the catheter is approached. The body jackets 160 can be reflowed over the underlying braid and allowed to flow through the interstitial spaces of the braid cells to bond the layers of the construct together. The jackets can be butted together in an axial series to form a continuous and smooth outer surface for the catheter shaft.

The distal tip section 210 can be subdivided into two or more regions, depending on the expansion characteristics allowed by the underlying braid. A proximal tubular section 211 can have largely the same profile and diameters as the elongate shaft 110 and transition into a low shear distal expansile section 213 designed to passively expand when a large, stiff clot is ingested. The tip section 210 can have one or more outer tip jackets 180, with the hardness of the jackets becoming progressively less as the distal end 214 of the section is approached to give the catheter a soft, atraumatic end.

The wires of the underlying braid can be wound in one spiral direction from the proximal end 212 of the tip section distally. Upon reaching a distal terminus, the wires can be inverted 238 to form distal hoops 230 and wind proximally in the opposite direction. As a result, the inverted ends are also more atraumatic as the free ends of the wires exist only at the proximal end 212 of the braid. This contrasts with, for example, other catheter designs where the braided reinforcing sections resemble a stent with wire free ends existing on both opposing ends of the device. The braid designs disclosed herein have the additional advantage of ensuring the sum of wires wrapped in one direction will necessarily be equal to those wound in the opposite direction, preventing any helical curl which could otherwise exist in the catheter as a result of an imbalance.

Differing braid patterns for the elongate shaft 110 and tip section 210 provide specific mechanical properties to the finished device. Some patterns can offer kink resistance and burst strength but can lack pushability. Other patterns offer greater torqueability and scaffolding support for the outer jackets at the cost of some flexibility. Still other patterns can give excellent flexibility and energy dispersion.

Additionally, other factors can be tuned as well. The catheter does not necessarily have to have two discrete braided sections for the shaft and distal tip. Three, four, or more discrete sections of differing flexibility can be used. Choices for other physical parameters, such as wall thickness and material composition, can also be implemented for the various catheter sections. For example, the stiffer and more proximal outer body jackets 160 can have a wall thickness 124 of approximately 0.004 inches to maintain column stiffness in the longitudinal direction without compromising to much lateral flexibility. More distal tip jackets 180 can have a slightly increased wall thickness 224 of approximately 0.006 to give the tip section an additional atraumatic cushion where very soft jacket materials are used. Ideally, there will be at least 0.0005 inches in wall thickness of jacket material radially outwardly of the braided structure and also radially inwardly of the braided structure where there is no liner.

Radiopaque marker bands can be included at different axial points along the length of the catheter 100 for visibility under fluoroscopy during a procedure. In the example illustrated, a marker band 118 can illuminate the proximal end 212 of the tip section 210 to give an attending physician an indication of where the expandable capacity of the catheter begins. The band shown can be platinum strip or other noble metal with a relatively short length of between approximately 0.025 - 0.030 inches and a thin wall thickness (approximately 0.0005 inches) to minimize the impact on flexibility and the outer diameter of the catheter.

In some examples, the band 118 can also provide the linking structure for the catheter 100 between the proximal end 212 of the braids of the tip section 210 and the distal end elongate shaft 110. This joint can allow different material and complex braid configurations to be used for the proximal and distal portions of the catheter and linked for a relatively low manufacturing cost and higher yields. The braids of the proximal elongate shaft 110 and tip section to be quickly manufactured separately to any of a number of desired lengths and patterns. If the catheter length is the typical 1320 mm of many designs, the tip section 210 can be approximately 100 mm in length 218, leaving a 1220 mm shaft length 130 terminating in a proximal luer. For more complex geometries or a more gradual stiffness transition, the tip section can be up to approximately 400 mm in length bonded with an 820 mm shaft at a more proximal marker band 118 location.

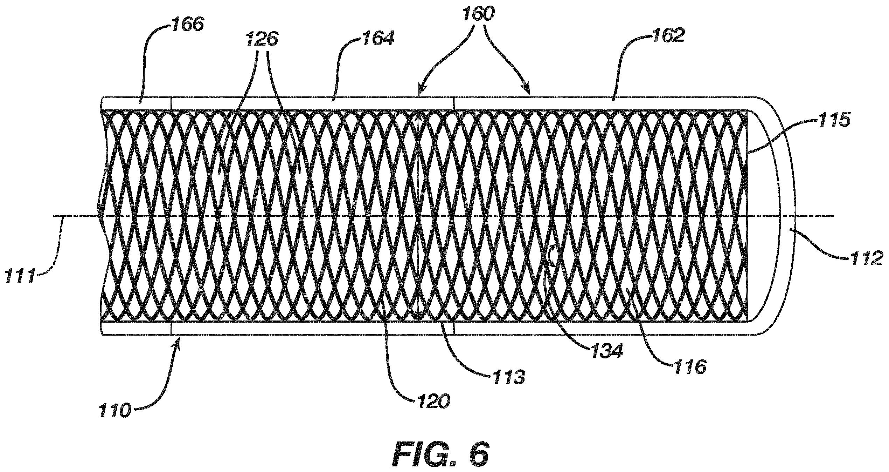

FIG. 6 depicts a cross section of the most proximal region of the elongate shaft 110. The plurality of braided segments 120 making up the support for the shaft can have a relatively dense weave with circumferentially elongated rings of cells 126 providing good scaffolding for the greater stiffness of the more proximal body jackets 160. The braid segments 120 can be dimensionally stable such that the half the wires are wound in one direction (for example, clockwise) while the other half of the wires are wound in the opposing direction. The structure can have other supporting members, such as helical coils extending underneath the braid, to resist tensile elongation and improve burst strength. An inner liner 115 can provide a low friction inner surface without any sagging or other structural protrusions into the interior lumen 116 during aspiration. In addition to or instead of the inner liner 115, hydrophilic coatings on one of both of the inner and outer surfaces of the shaft can also be used.

The kink resistance of the shaft is in part due to the structure of the braided segments 120 in cooperation with the outer polymer body jackets 160. Adjusting the braid PPI, pitch, and cell angle, combined with pre-stretching or shortening other liner and/or outer jacket materials, can effectively set the longitudinal stiffness and the force required to bend different sections of the shaft. The braided segments 120 of the proximal shaft can have a PPI in a range from approximately 100 to approximately 170, or where a coil is placed underneath, a PPI of 60 can be used as the coil will improve the kink resistance of the 60PPI braid while taking advantage of the improved flexibility of the lower braid PPI. Braid PPI tends to be stiffest below 60PPI, most flexible at 60 PPI, and gradually stiffer above 60PPI, although higher PPI values of 170 would still be more flexible than a 40PPI braid. Kink resistance improves with higher PPI. The proximal braid angle 134 in this region of the shaft can be in a range from approximately 140 degrees to approximately 170 degrees.

Torqueability and kink resistance can be gained by using stainless steel wires in a diamond two-over-two pattern where two wires side by side alternately pass under two wires, then over two other wires. A herringbone pattern, or flattened wires, can also be utilized for a lower profile cross section. For example, flat braids can reduce the profile while offering a similar stiffness to a round braid of the same cross sectional area.

The jackets 160 can transition distally to progressively softer materials in a stepwise fashion for added flexibility. By way of example and not limitation, the shaft portion shown can have a most proximal jacket 162 which can span a significant distance (approximately 1000 mm) from the proximal end 112 and be Pebax, TR 55, ML 21, or similar material having a hardness of approximately 72 Shore D. The next, more distal jacket 164 can span approximately 60 mm and be composed of Pebax or similar elastomer with a hardness in the range of 63D. The third jacket 166 can extend a further 50 mm and be Pebax of about 55D. Jackets from the proximal and most distal end of the catheter can include hardness's of between 85D near the hub and 20A at the tip.

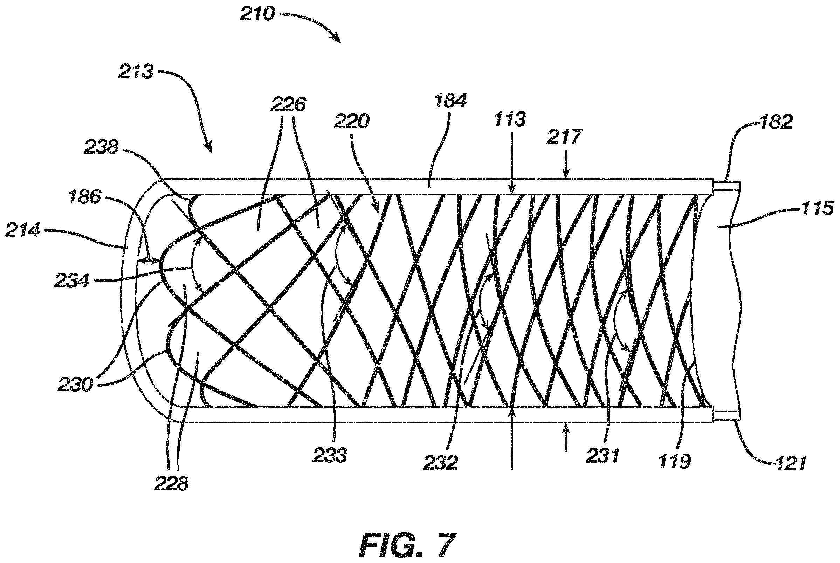

A magnified partial cross section view of an example internal configuration for the low shear tip section 210 of the large bore catheter is depicted in FIG. 7. The more proximal tubular section 211 and the distal expansile section 213 of the section can have approximately the same nominal inner diameter 113 as the elongate shaft 110. The plurality of braids 220 can provide good scaffolding for the outer tip jackets 182, 184, especially in regions where a particularly soft and highly flexible polymer or blend with less structure is chosen.

The wires of the braided sections 220 of the tip can be Nitinol, DFT, or another shape memory superelastic alloy for improved flexibility and robustness when deployed in tight bends (the use of DFT wire can also give the tip section radiopaque qualities while maintaining shape memory characteristics). The looped ends can also include radiopaque sleeves or coils to enhance visibility. The braid can thus have an inner diameter 113 the same as that of the catheter bore and have a constant nominal outer diameter 217 so that the catheter 100 can be advanced through an outer catheter with an inner diameter that is at least 0.002 inches greater. An added benefit of using a superelastic material for the braid wires is that the catheter walls can be maintained relatively thin for the tip section 210 without sacrificing desirable performance characteristics such as flexibility or crush strength and that the catheter lumen can recover if subjected to forces that cause it to kink.

As discussed previously, the braid wires can invert 238 proximally back upon themselves as shown to form distal hoops 230 at the distal end 214 of the tip braid sections 220. This forms the braid in a one over one half-diamond pattern where a single wire passes under, then over another single wire. Fewer wires can thus be used as each individual wire will form a hoop 230. The hoops eliminate any potential of sharp braid ends migrating through the polymer encapsulation of the outer jackets during use. Two or more wires can also be tied together as one for additional reinforcement in the braid.

It is possible that the entire catheter supporting braid (i.e. both the elongate shaft 110 and tip section 210) can be a single monolithic braid of one material extending from the proximal end 112 to the distal end 214 with looped distal hoops 230. However, it is difficult to manufacture the hoops 230 with a braid that is greater than 400 mm in length with reasonable yield rates. It can be more feasible to manufacture a distal tip section 210 having braids 220 that are at most 400 mm long and join to simple proximal braids 120 of the elongate body at a joint such as the proximal marker band 118 as in FIG. 5. Nitinol braid wires like those of the tip can be used for the proximal braids, but less expensive stainless steel wires can perform in these regions for axial stiffness and with less cost.

The plurality of braids 220 making up the tip section 210 can form circumferential rings of closed cells 226. The cells 226 can deform as the braid wires slide relative to one another and are capable of elongating circumferentially with respect to the axis 111 when the distal expansile section 213 is radially expanded from the forces of ingesting a clot, and elongating axially (flattening) with respect to the longitudinal axis when the expansile section is reduced regains its shape when the clot has passed through. The cells 226 can be substantially diamond shape to allow the vertices to facilitate more uniform deformation/expansion as shown or take some other profile if the braid wires follow a non-linear pattern.

The crossover of the braid wires form braid angles 231, 232, 233, 234 at the vertices of each of the cells 226, which help define the expansile capabilities of different portions of the tip. Variations in the cell angles allow for different levels of expansion for the tip during clot aspiration. Generally, the braid angles can become smaller and more acute as the distal end 214 is approached to aid in interacting with and expanding to receive a clot.

Nominal angles can be chosen to balance the desired competing capabilities of the tip (delivery and target site performance). In general, smaller braid angles (for example, less than 125 degrees) yield greater expansion capability to conform to the contours of a large or firm, fibrin-rich clot as it is ingested. This added expansion allows for better clot management and reduces the risk of shearing when compared to other tips with stiffer, less compliant frames or those utilizing stiffer polymeric materials. Further, smaller angles offer less resistance to collapse and better force transmission when transiting through an outer guide sheath. However, these characteristics can come at the cost of some flexibility and radial force to resist tip collapse during aspiration.

Alternately, high braid angles (where the wires are aligned more radially) can provide better compressive hoop strength in an expanded state to resist collapse of the tip under aspiration. More obtuse cell angles can also limit the ability of the expanded tip to over-expand radially in compression when the catheter 100 is being advanced through a vessel independently (after deployment from the guide sheath). This can help the expanded tip avoid snagging in vessels, particularly in tight bends and when being pushed through vessels of progressively smaller diameters.

By way of example, a first most proximal braid angle 231 can be approximately 150-160 degrees. Additional more expansile capability can be incorporated by transitioning to more distal braid angles 232, 233 of approximately 105 degrees and 100 degrees, respectively. a distalmost final braid angle 234 can be between 65-95 degrees.

A distal marker band 121 approximately 0.8 mm in length can be included just proximal of the distalmost tip jacket 184 to give radiopacity near the distal end 214. The marker band 121 can be platinum or another suitable noble metal and can be crimped over the axial spine (not shown) and the braid 220 of the tip section can extend over the band. The band 121 can also be situated at or near the distal end 119 of the inner liner 115 approximately 5 mm and up to 10 mm from the distal end 214 of the tip section 210. In cases where a very short tip is desired for increased trackability, this distance can be shorter (approximately 2 - 3 mm).

To be compatible with many of the most widely adopted guides and/or sheaths, the nominal inner diameter 113 of the catheter elongate shaft 110 and unexpanded tip section 210 can be sized and scaled appropriately. For example, a 5Fr catheter targeting vessels approximately 2.0 mm in diameter can have an inner diameter 113 of approximately 0.054 inches. Similarly, a 6Fr catheter targeting vessels approximately 2.3 - 3.4 mm in diameter can have an inner diameter 113 of approximately 0.068 - 0.074 inches. A larger 8Fr catheter for less remote clots can have an inner diameter 113 of approximately 0.082 - 0.095 inches. Theoretically, there is no upper bounds for the inner bore diameter, but in practice it is preferrable to keep the groin puncture size below 10Fr.

To create a more atraumatic vessel crossing profile, the distalmost tip jacket 184 can extend for a distance 186 to overhang beyond the distal hoops 230 of the tip section braids 220. The distance can be in a range of approximately 0.1-1.0 mm or, more preferably, 0.5-0.8 mm. The jacket 184 can be the softest of those on the catheter and can cover up to approximately the distal 90 mm of the catheter length. The ultimate softness of the jacket must be balanced with the crush resistance and expansile behavior of the braid. In one example, Neusoft with a hardness of approximately 40 Shore A can be reflowed to form the distalmost tip jacket 184. In some cases, an even softer layer of approximately 20 - 40 Shore A can be used. The next proximal outer jacket 182 can be Pebax with a hardness in the region of 25 Shore D.

In some examples, to allow for smooth delivery inner surfaces of the catheter lumen can be coated with a low-friction or lubricious material, such as PTFE or commercially available lubricious coatings such as offered by Surmodics, Harland, Biocoat or Covalon. This coating facilitates the passage of auxiliary devices and aids with a captured clot being drawing proximally through the catheter with aspiration and/or a clot retrieval device.

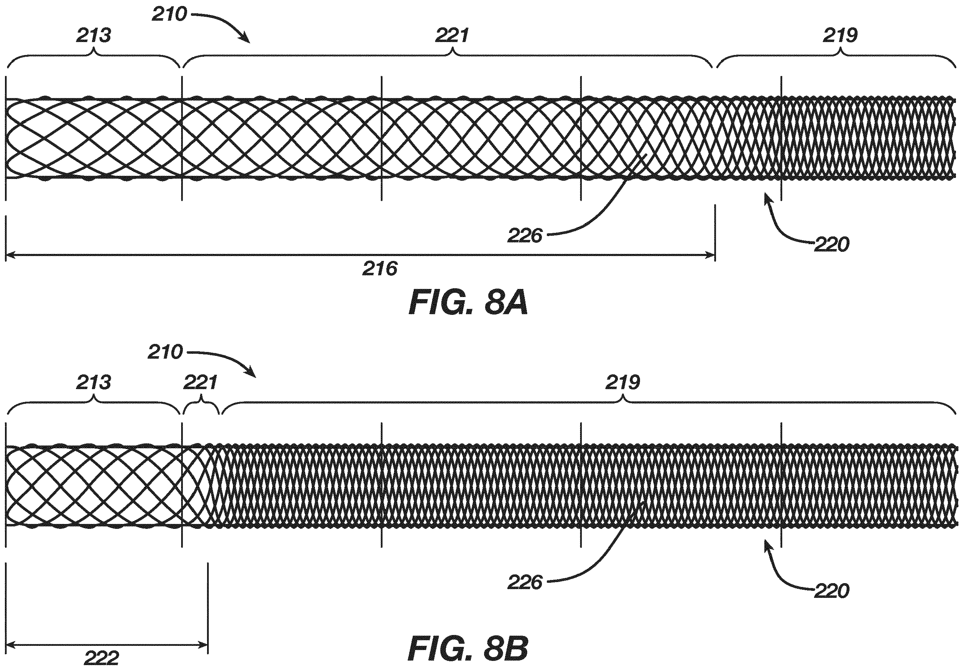

The examples illustrated in FIG. 8A and FIG. 8B show how the plurality of braided sections 220 making up the low shear tip section 210 can have variable PPI and braid angles. Sixteen Nitinol wires in the one over one half-diamond pattern can be used to produce eight distal hoops 230 (two wires grouped together as one for strength). In another example, twelve distal hoops can be created using 24 wires. As seen, more proximal sections can have a greater PPI than more distal regions. Generally, the PPI of the braid will have an inverse relationship with the expandability of a particular section of the tip 210. Regions with a larger PPI can have greater flexibility but limited expansion capability. Similarly, regions with lower PPI values can trade hoop strength for markedly greater expansion capability.

The braided sections 220 can sub-classified into segments by the braid characteristics and their contribution towards the expansion capabilities of that segment. The braid in a most proximal section 219 can be the "stiff or "more proximal" section as described herein. An intermediate expansile section 221 can alternately be desired as a "intermediate" or "mid" section. Similarly, the distal expansile section 213 with the greatest radial expansion capability can be referred to as the "least stiff' or "more distal" section.

The distalmost cells 228 can have the smallest cell angle corresponding to the greatest expansion capability. This is consistent with some of the qualities of the low shear tip, which can prioritize successfully ingesting a clot so that it can be protected during the rest of a procedure, even if the clot cannot be fully aspirated immediately. FIGs. 8A and 8B illustrate final cell angle of 65 and 95 degrees, respectively. Generally, it has been found that braid angles of 110 degrees or lower gives radial expansion under compression. Therefore, the size of the expansion zones can be tailored by changing the length at which angles of 110 degrees or lower are maintained proximally. Alternatively, the expansion zone can have a variable braid angle over a length.

Designs can have expansile sections with axial lengths 216 that are relatively long for more accommodating clot management characteristics. FIG. 8A shows the braid angles of the distal expansile section 213 increasing proximally to approximately 154 degrees over a length 217 of the distal 213 and intermediate 221 expansile sections. A longer intermediate expansile section 221 can have a progressive increase in cell angle (for example to 105 degrees and eventually to 154 degrees) and PPI (from approximately 40 up to 140) proximally to allow an ingested clot to be gradually compressed as it transits the section. Longer sections, however, can be less flexible and more prone to elongation or shortening under axial loads as the clot moves proximally. Alternatively, the example in FIG. 8B exhibits a much more rapid transition over a substantially shorter expansile length 222 of the tip. A short length 222 for the expansile section can improve hoop strength and resistance to collapse under aspiration.

It should be noted that regardless of the specific design of the tip section, any of the herein disclosed catheters designs can also be used with one or more stentrievers. The combined stentriever retraction and efficient aspiration through the large catheter bore can act together to increase the likelihood of first pass success in removing a clot. The catheter can also direct the aspiration vacuum to the clot face while the stentriever holds a composite clot (comprised of friable regions and fibrin rich regions) together preventing embolization and aid in dislodging the clot from the vessel wall. The stentriever can help stabilize and urge the clot proximally as it is compressed in the catheter.

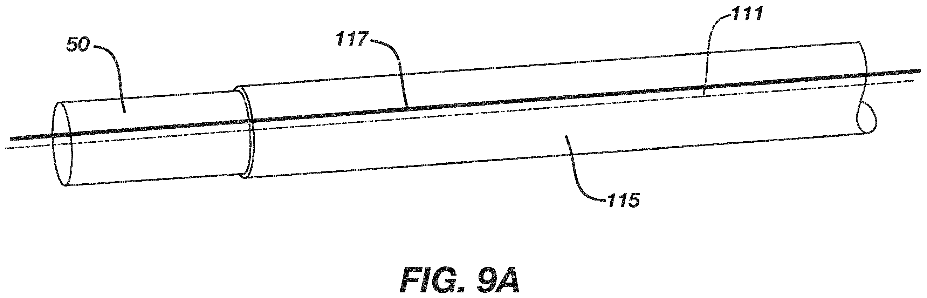

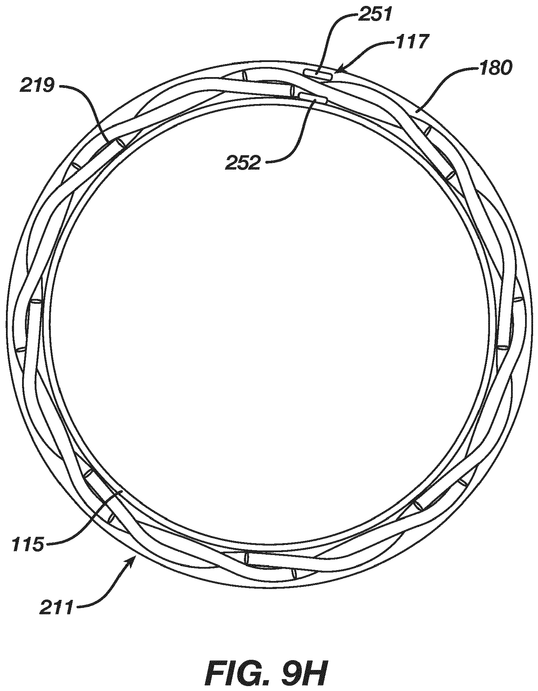

A method for manufacturing a catheter utilizing the disclosed designs is illustrated in FIGs. 9A-9H. FIG. 9A shows a low friction liner 115 positioned on a supporting application mandrel 50. For a typical 6Fr catheter, the mandrel can have an outer diameter of approximately 0.071 inches. The mandrel can often be silver-plated copper (SPC) as is commonly used for these applications. Alternatively, especially ductile materials (such as PEEK) can be used which stretch to neck down from a nominal outer diameter so that the mandrel can be removed without excessive friction after completion of the catheter assembly. Further mandrel materials can include nylon coated copper or nylon coated steel.

The inner liner 115 can be a lubricious, low friction material such as film cast PTFE with a very thin wall thickness (0.00075 inches) and can include an etched surface and/or outer tie layers to help with bonding. The liner can aid in a clot being pulled proximally through the catheter with aspiration and/or a clot retrieval device such as a stentriever. The liner can also help with the initial delivery of any associated devices to the target site through the lumen of the catheter. Depending on the material chosen, it can also be stretched to alter the directionality of the liner material (e.g. if the liner material has fibers, an imposed stretch can change a nominally isotropic sleeve into a more anisotropic, longitudinally oriented composition) to reduce the wall thickness as required.

An axial spine 117 can be positioned along the tie layer beneath the braid to counteract any tensile elongation of the shaft. The spine can be of a threaded formulation such as Zylon or another LCP so that it is not stiff in compression and can be flattened beneath the braid for a reduced cross sectional profile. An LCP can offer the highest tensile strength, while a stainless steel spine or Nitinol can offer the best pushability at the cost of some lateral flexibility. As another option, some of the LCP spine can be replaced with a stainless steel and/or Nitinol spine.

Initially, a length of the spine can extend distally well beyond the distal ends of the inner liner and application mandrel as illustrated to aid with further assembly steps during manufacturing. Other examples can use additional spines, such as two spines spaced 180 degrees apart, for added tensile strength. Wrapped ePTFE liners can also be used to reduce tensile elongation while maintaining excellent lateral flexibility.

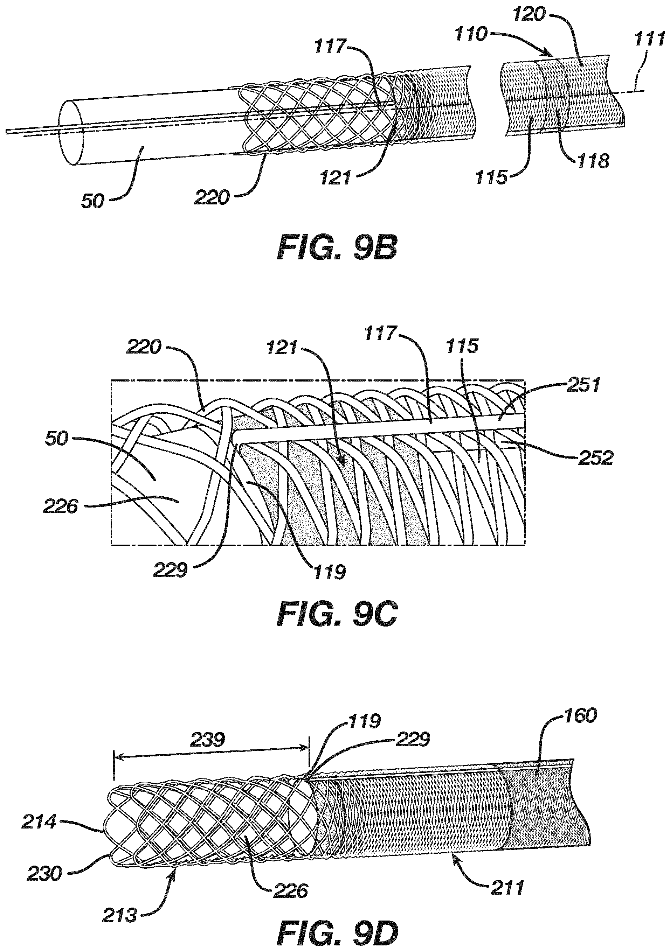

A braided backbone 120 of the elongate body shaft 110 is positioned around at least a proximal portion of the inner liner 115 and application mandrel 50 in FIG. 9B. The braids can extend distally from a luer (not shown) on the proximal end of the catheter to overlap with a radiopaque proximal marker band 118 positioned proximal to the distal end of the inner liner 115. A distal marker band 121 can also be crimped into place at or near the distal end of the inner liner before or after application of the spine.

A plurality of braids 220 supporting the low shear tip section 210 is threaded proximally over the application mandrel 50 and inner liner 115 using the distal portion of the spine 117 as a guide. In some examples, the braids 220 of the tip section can be cut from a wires of Nitinol or another shape memory superelastic alloy for additional toughness and flexibility. The tip section braids 220 can have at least a nominal inner diameter and nominal outer diameter approximately the same size as the braids 120 of the shaft.

The plurality of braids 120 around the shaft 110 can overlap with the proximal marker band 118 at their distal end. Similarly, the most proximal portion of the tip section braids 220 can overlap at their proximal end with the band 118. Both overlapping braids can be disposed over the spine 117 and welded to the marker band 118. In some instances, the braided sections can be woven together for additional strength prior to being welded. In other instances, an adhesive can be used in addition to, or instead of, the welded joint. Alternately, the braided sections could be welded together directly, or incased in a more proximal (approximately 200 mm to 300 mm proximal of the tip) polymer jacket of high tensile strength. This jacket can be, for example, Pebax 25D or Pebax 72D depending on the desired final stiffness of the catheter.

Though the proximal braid 120 of the shaft 110 can also be produced from Nitinol, the use of a mid-joint for the braids at the proximal marker band 118 allows it to be manufactured from stainless steel or similar material for increased axial stiffness and reduced cost. This gives the advantage of allowing more complex design features in the distal Nitinol braid of the tip section 210 to join with lower cost standard proximal braid and/or coil sections at the band 118. This flexibility increases manufacturing yields through the separation of complex and standard catheter shaft sections. The use of specific metallics such as platinum (which can be welded to both stainless steel and Nitinol) for the sleeve of the marker band 118 can replace the use of adhesives or other means and create a more robust joint. The band can be kept relatively short, for example between 0.3-1.0 mm, in order to minimize the impact on shaft flexibility.

The axial thread of the spine 117 can be pulled through a distal braid opening of a cell 226 the tip section 210 and looped proximally to connect the spine more securely to the distal region of the catheter, as shown in FIGs. 9C-D. The loop 229 can create both an outboard spine portion 251 extending atop the braid pattern 222 and an inboard spine portion 252 running beneath the braid. The loop 229 can be positioned more proximally, such as at a distance 239 from the distal end 214 of the tip, or at the distal end 119 of the inner liner 115 as seen in FIG. 9D. Alternatively, the loop 229 can be at a more distal location up to or through the distalmost cells 228 of the tip section 210. The distance for which the spine 117 is looped back on itself can be kept short (<5 mm) or can be a much greater distance of approximately 50 mm. In many examples, after inverting at the loop 229 the spine 117 can return all the way to the proximal end of the catheter shaft.

FIG. 9D shows that once the spine has been looped back, an axial series of separate outer polymer jacket extrusions 160 can be reflowed or laminated in place on the elongate shaft 110. The applied heat can allow the outer polymer to fill the interstitial sites between the braid of the elongate body and around the spine 117. This flow can also help to fix the jackets axially so they cannot slide distally. Depending on their axial location on the elongate body 110, the jackets 160 can alternately be sprayed, dipped, electro spun, and/or plasma deposited. The jackets can preferably be in an axial series, but some combination of axial and radial series can also be used. Transitions between jackets segments can include angled cuts for a more gradual transition of stiffness

Suitable jacket materials can include thermoplastic elastomers like those of the Pebax family which can have a wide range of mechanical and dynamic properties. The jackets 160 can have differing durometer hardness and/or wall thicknesses. For example, a stiffer more proximal portion of the shaft can have a jacket with a thickness of approximately 0.004 inches and a hardness of around 72D. By contrast, a distal section requiring greater flexibility but where the underlying support braid is less dense can have a jacket with a wall thickness in the region of 0.006 inches and a hardness of around 40A. With the body jackets in place, the application mandrel can be removed from the assembly.

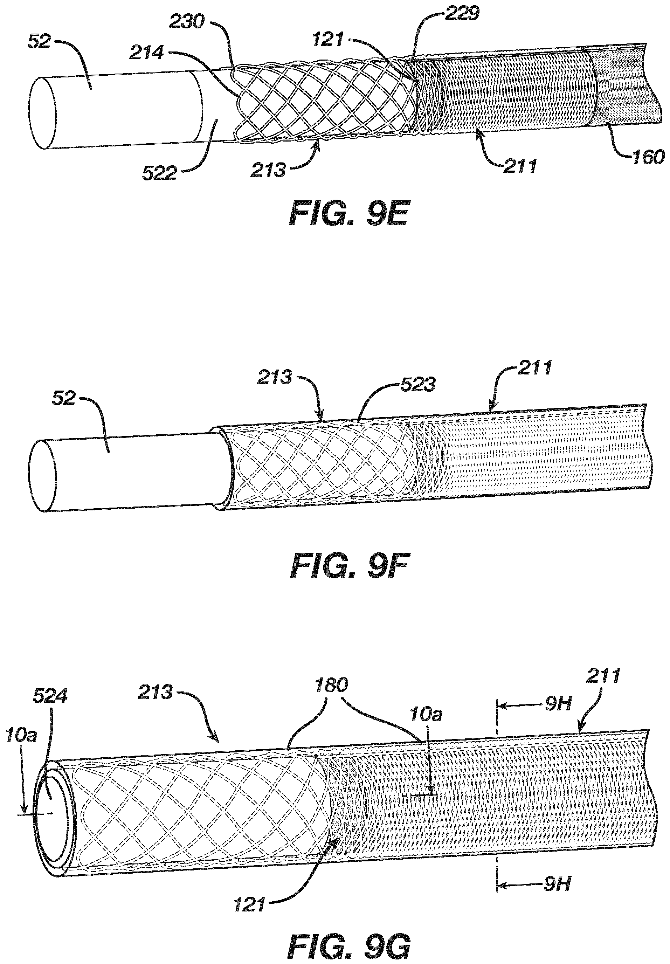

FIG. 9E depicts a reflow tool 52 with an inner distal jacket 522 applied over at least the portion of the tool loaded proximally into the distal end 214 of the assembly. The supporting braided frame of the distal tip section can allow very soft jacket materials to be used here for their atraumatic properties, such as Neusoft with a hardness below 30 Shore D (approximately 80 Shore A). The inner jacket can be thin (e.g. approximately 0.003 inches) and is used to gain more uniform wall thickness for the tip section and ensure complete encapsulation of the braids so the lumen is smooth and unobstructed. A soft, elastic outer polymer tip jacket 184 extrusion can be threaded or stretched over the outer surface of the reflow tool 52 and at least a portion of the distal expansile section 213 and tip proximal tubular section 211 (FIG. 9F). Alternatively, the outer distal jacket 523 material can be sized to fit loosely over the reflow tool 52 The outer distal jacket wall thickness can be 0.003 inches such that the final wall thickness of the combined distal polymeric jacket is approximately 0.006 inches. The jacket can extend proximally to the distal edge of the proximal outer jackets of the elongate body and overhang at least several millimeters distally beyond the distal end of the braid hoops 230 of the distal tip braid. FIG. 9G illustrates the inner jacket layer 522 and outer jacket layer 523 reflowed to the tip section to fuse as a homogenous distal tip jacket 180.

In some instances, a temporary sleeve of PTFE or other suitable low friction material can be applied over the pre-reflowed shaft and used to control and arrest the proximal flow of the distal tip jacket 180 as it is reflowed into place. In other examples, a layer of heat shrink can be positioned around the outer jacket 180 extrusion to better conform the material to the contours reflow tool 52 during reflow or lamination. Once complete, reflow tool 52 can be removed from the assembly, and if necessary, any excess material can be trimmed away to ensure the desired distal profile of the catheter is attained. When removed at least the distal 20 cm of the inner surface distal of the inner liner termination can be coated with a hydrophilic coating 524. A process such as dip coating can be used to for the application of this coating.

FIG. 9G shows a cross section view through a portion of the completed tip proximal tubular section 211 proximal of the distal marker band 121. The proximal tubular section braid 219 can have a pattern with properties resembling those discussed earlier. The spine 117 can invert proximally and loop back such that an outboard section runs 251 atop the braid and an inboard section 252 runs beneath the braid. These segments can swap places if the spine is woven through the openings in the braid during manufacturing.

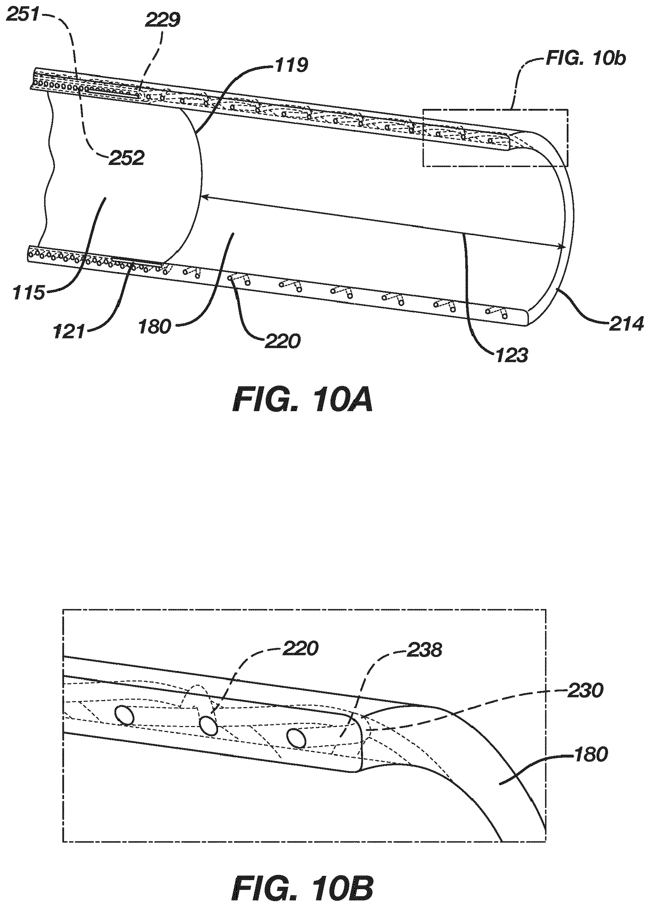

A cross section view of through a longitudinal section of the tip section is illustrated in FIG. 10A. The distal end of the inner liner 115 can be positioned a distance 123 from the distal end 214 of the tip. This distance can frequently be approximately 5 mm but can be up to 25 mm from the distal end 214. The spine loop 229 can be seen inverting near the distal end of the inner liner 115 and distal marker band 121 to form the outboard 251 and inboard 252 sections.

The second plurality of braids 220 can be encapsulated within the tip jackets 180 and grow considerably less dense as the distal end 214 is approached. A magnified view of the braid inversions 238 to form the distal hoops 230 of the braid is presented in FIG. 10B. The hoops 230 can be broad and atraumatic enough so as to not to risk a puncture of the jackets 180 under radial expansion or in tight bends in the vasculature. The inversion allows the wires to be spiral woven in the opposite direction proximally to 1 over 1 under pattern

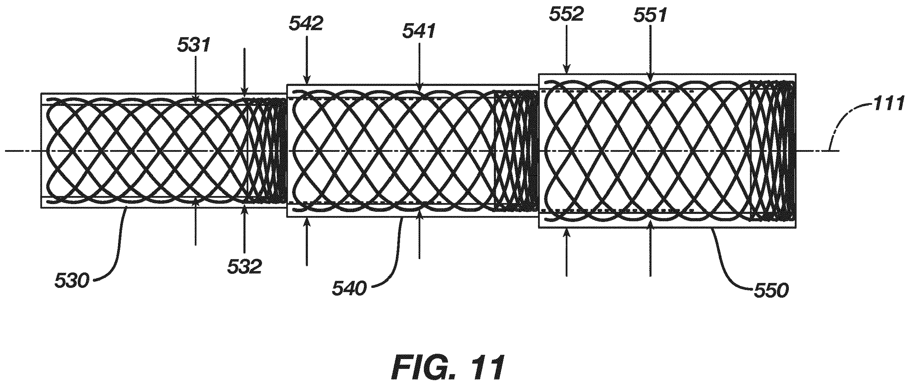

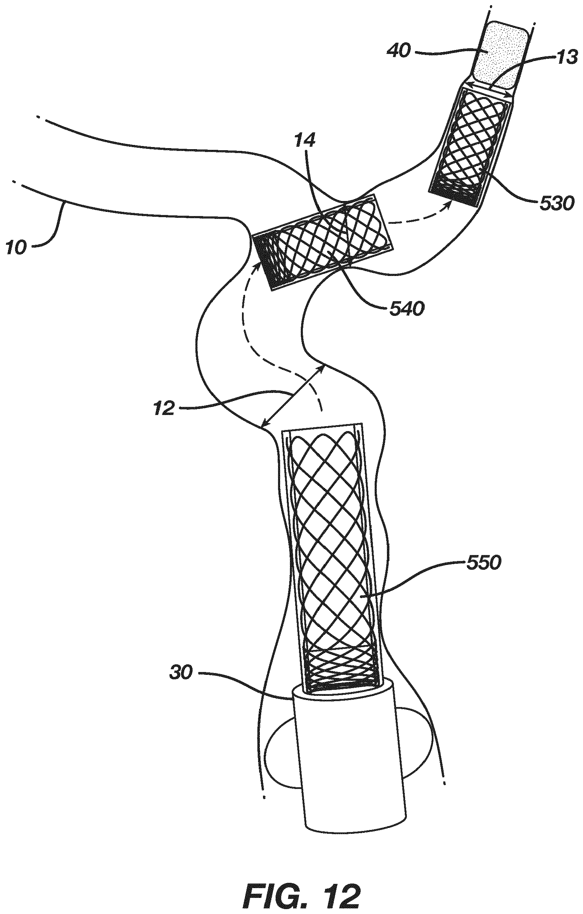

When using a catheter of the present disclosure to clear an occlusion from a body vessel, the large bore catheter with a low shear tip can be delivered through one or more outer catheters to a location proximal of a vessel occlusion. A telescoping system using outer catheters of stepped diameters is demonstrated in FIG. 11 and FIG. 12. Preferably, each catheter in the system has a low shear tip section as described herein so that when a vessel occlusion is finally reached, the appropriately sized catheter can have the best chance of adapting to and successfully swallowing the clot. Additionally, if a firm clot becomes stuck in the lumen of an inner catheter, the outer catheters can be used as a conduit for withdrawal. As such the catheters can be supplied to be 3Fr, 4Fr, 5Fr, 6Fr, 7Fr, 8Fr, and 9Fr compatible or greater. Offering a range of catheter sizes allows to user to choose a size that fits most optimally with a particular patient presentation.

Ideally, for Neurovascular aspiration, the catheters can be offered with 5Fr to 9Fr compatibility to cover the relevant vessel size range and to keep groin puncture size as small as possible (below 10Fr). Large French size catheters offer more flow rate through the lumen of the catheter but can be stiffer in nature and are often more difficult to advance distally in tortuous vessels, especially up to M2 (middle cerebral artery) locations. In the example of a middle cerebral artery occlusion, an outer catheter or guide sheath might be placed in the internal carotid artery proximal of the carotid siphon. If for example the target occlusion is in an M1 vessel, one or more of the outer catheters will likely need to be maintained in a position well proximal of these vessel diameters.

FIG. 11 shows a system where an innermost aspiration catheter 530 is being used with two outer aspiration catheters 540, 550 concentrically around the longitudinal axis 111. In an example, the inner most aspiration catheter 530 can be 5Fr, the next outer aspiration catheter 540 can be 6Fr, and the final outer aspirating catheter can be 8Fr. This example can have approximately the dimensions in the following table:

| **Catheter Type** | **Catheter ID (*in*)** | **Catheter OD (*in*)** | **Target Vessel ID (in)** |
|---|---|---|---|
| **5Fr Low Shear Tip** | 0.054 | 0.066 | > 0.067 |
| **6Fr Low Shear Tip** | 0.068 - 0.074 | 0.080 - 0.086 | 0.081- 0.087 |
| **8Fr Low Shear Tip** | .082 - 0.095 | 0.094 - 0.115 | 0.095 - 0.116 |

Ideally, the set of catheters is sized such that there is approximately 0.001 - 0.006 inches of clearance between the outer diameter of any given catheter and the inner diameter of the next larger catheter. This minimizes any steps which can occur between the OD and ID of two consecutive catheters in the series when tracking through vessels so as to not snag on vessel side branches or other obstructions for a smooth crossing profile.

FIG. 12 illustrates how this range of catheter sizes can be used simultaneously in a telescoping fashion within the vasculature 10 to reach a clot 40. The user can advance at least two catheters together in a telescoping fashion to the treatment location with the smallest inner catheter 530 size chosen for the vessel diameter 13 at the target site. Progressively smaller vessel diameters 12, 14 can be encountered along the path. If the clot is located in a vessel with a larger inner diameter than that of the inner catheter 530, the tip of the inner catheter can expand safely within the vessel as the clot is ingested so that it creates an aspiration seal with the vessel or leaves a small clearance between the vessel and the catheter tip. The larger sized outer catheters 540, 550 can serve as backups should a stiff clot become lodged in the shaft of the inner catheter 530, which can then be retracted through the shaft of the next catheter.

For example, if a larger diameter catheter size is chosen to reach a target site but is incapable due to tortuosity, a smaller diameter catheter 530 can be advanced through the lumen of the larger catheter 540 to travel more distally and reach the treatment location. For example, if a 6Fr catheter 540 is used to reach a clot in the proximal M2 but can only navigate as far as vessels in the proximal M1, a 5Fr catheter 530 can be advanced telescopically to reach the proximal M2. The 6Fr catheter 540 can maintain access so that the 5Fr catheter 530 can be quickly advanced. After the clot 40 has been removed with the 5Fr catheter, the 6Fr catheter can be used to check vessel patency and if any fragments remain, they can be aspirated through the 6Fr catheter or through co-aspiration with a clot retrieval device/stentriever.

When used with a clot retrieval device, stiff clots which cannot be fully ingested into the catheter lumen can be locally held in place by the clot retrieval device in the catheter tip. The device, clot, and inner catheter can then be drawn into the inner diameter of an outer catheter for retrieval or into successively larger catheters as necessary. The clot can be stabilized and scaffolded by the clot retrieval device during this procedure.

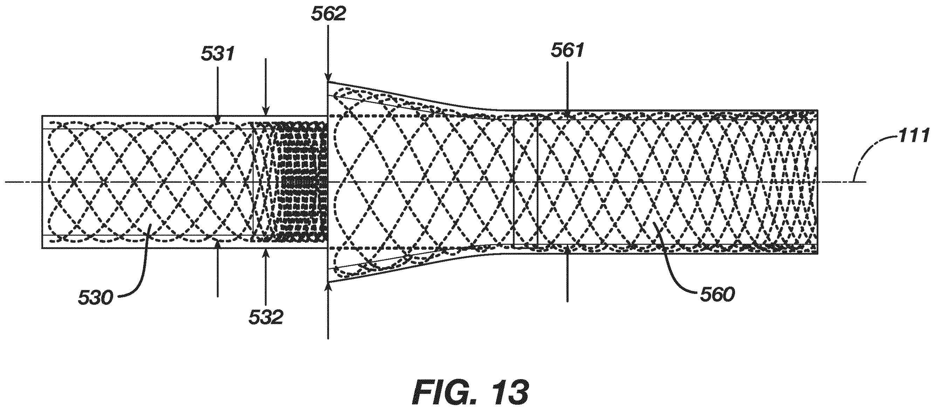

In some cases, one or more of the outer catheters in the system can be a collapsible super-bore funnel catheter 560, or a large bore catheter with a self-expanding tip, as depicted in FIG. 13. When deployed from an outer catheter, the self-expanding tip can expand radially to form a funnel shape with a larger inner diameter 561 for aspiration prior to ingesting a clot 40. In many cases, the funnel shape can be sized to seal with a vessel of similar size to block fluid proximal of the super-bore tip from being drawn into the self-expanding tip during aspiration.

Similar to earlier telescoping examples, if a super-bore funnel catheter 560 is chosen to reach a target site but cannot reach that location due to tortuosity, a smaller, constant outer diameter catheter 530 with a low shear tip can be advanced through the lumen of the super-bore catheter to travel more distally and reach the treatment location.

For example, if an 8Fr collapsible super-bore catheter with a funnel inner diameter of 0.140 inches is used to aspirate and retrieve a large clot in the Internal Carotid Artery and branches (or a 6Fr catheter in the M1 vessels), the user then injects contrast to check vessel patency. If the user finds that some clot fragments remain in more distal vessels, the collapsible super-bore catheter can be advanced to aspirate the fragments in the more distant location. If access cannot be achieved due to tortuosity, or if the funnel outer diameter has reached a point where it is approximately equal to the inner diameter of the vessels, an inner catheter with a smaller diameter 6Fr (or 5fr) low shear tip can be rapidly advanced telescopically to the more distal vessel location to retrieve the clot through aspiration or co-aspiration with a stent retriever. As the inner catheter and clot is retracted through the super-bore catheter, the plunger-type suction effect of retracting one within the other can act to aspirate through the mouth of the super-bore catheter to ensure that any fragments that may have dislodged from the initial clot retrieval process are retrieved. Aspiration can also be directly applied to the lumen of the super-bore catheter so that a negative flow through it can be maintained during the procedure.

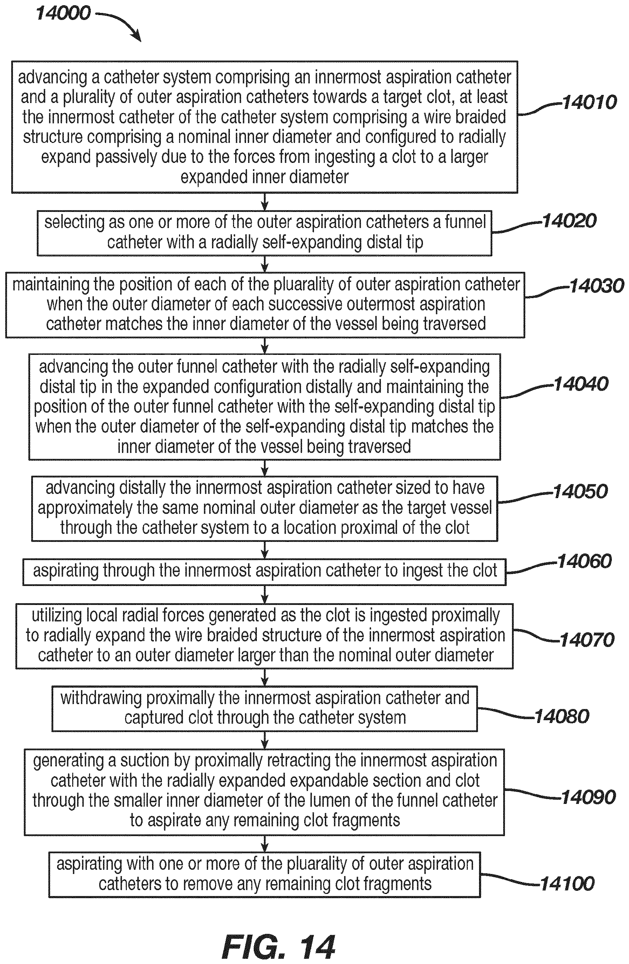

FIG. 14 is a flow diagram showing method steps for removing a clot in a vessel using a catheter with an expansile low shear tip of the present disclosure. The method 14000 can have the step 14010 of advancing a coaxial catheter system comprising an innermost aspiration catheter and a plurality of outer aspiration catheters towards a target clot. The innermost catheter can be sized to have an outer diameter roughly the same as the predicted inner diameter of the target vessel. The catheters in the system can have low shear tips like those described herein capable of passive expansion when impinged by a large, stiff clot being aspirated.

In step 14020, at least one of the outer aspiration catheters can be a super-bore funnel catheter with a large lumen and a radially self-expanding distal tip. The funnel tip can have an outer diameter capable of sealing off proximal fluid in a vessel and progressively compressing a clot as it is drawn into the tip. The position of each successive aspiration catheter in the system can be maintained when the nominal outer diameter of each approximates the inner diameter of the vessel being traversed so that the vessels are not damaged (step 14030). At least two of the catheters can be advanced to a location proximal of the target site so that if a stiff clot becomes lodged in the lumen of the inner catheter, the outer catheter can serve as a backup for the inner catheter and captured clot to be withdrawn through.

The funnel catheter can be advanced to aspirate the target clot. Should the outer diameter of the expanded tip reach the inner diameter of a vessel being traversed, a smaller diameter inner catheter with a low shear tip can be directed distally through the lumen to reach the target site in steps 14040 and 14050. The clot can be aspirated into the mouth of the inner catheter, which can allow some radial expansion in step 14070 through interaction with firm portions of the clot to cover and secure it from snagging or shearing.

As the inner catheter and captured clot are withdrawn into the outer funnel catheter, the expanded section of the inner catheter can in some cases be equal to or slightly larger than the inner diameter of the larger outer funnel catheter. This means that retracting the inner catheter through the funnel catheter can create a suction to aspirate through the mouth of the funnel catheter as space is vacated by the inner catheter (step 14090). The aspiration can draw any fragments from the clot through the lumen of the funnel catheter and prevent downstream migration. If necessary, aspiration can also be directed through one or more of the other outer catheters in the system if distal embolization is a concern.

The invention is not necessarily limited to the examples described, which can be varied in construction and detail. The terms "distal" and "proximal" are used throughout the preceding description and are meant to refer to a positions and directions relative to a treating physician. As such, "distal" or distally" refer to a position distant to or a direction away from the physician. Similarly, "proximal" or "proximally" refer to a position near or a direction towards the physician. Furthermore, the singular forms "a," "an," and "the" include plural referents unless the context clearly dictates otherwise.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 99%.

In describing example embodiments, terminology has been resorted to for the sake of clarity. As a result, not all possible combinations have been listed, and such variants are often apparent to those of skill in the art and are intended to be within the scope of the claims which follow. It is intended that each term contemplates its broadest meaning as understood by those skilled in the art and includes all technical equivalents that operate in a similar manner to accomplish a similar purpose without departing from the scope and spirit of the invention. It is also to be understood that the mention of one or more steps of a method does not preclude the presence of additional method steps or intervening method steps between those steps expressly identified. Similarly, some steps of a method can be performed in a different order than those described herein without departing from the scope of the disclosed technology.
Aspects of the invention:
1. A method for treating a clot in a target vessel, the method comprising the steps of:
   advancing a catheter system comprising an innermost aspiration catheter and a plurality of outer aspiration catheters towards the clot, at least the innermost aspiration catheter of the catheter system comprising:
      a wire braided structure comprising a nominal inner diameter and configured to radially expand passively due to forces from ingesting the clot to a larger expanded inner diameter;
   position of each of the plurality of outer aspiration catheter where an outer diameter of each successive outermost aspiration catheter is approximately equal to an inner diameter of a vessel being traversed;
   advancing distally the innermost aspiration catheter sized to have a nominal outer diameter approximately equal to an inner diameter of the target vessel telescopically through the catheter system to a location proximal of the clot;
   aspirating through the innermost aspiration catheter to ingest the clot;
   utilizing local radial forces generated as the clot is ingested proximally to radially expand the wire braided structure of the innermost aspiration catheter to an outer diameter larger than a nominal outer diameter of the innermost aspiration catheter; and
   withdrawing proximally the innermost aspiration catheter and captured clot through the catheter system.
2. The method of aspect 1, further comprising the step of selecting as one or more of the outer aspiration catheters a funnel catheter with a radially self-expanding distal tip.
3. The method of aspect 2, further comprising the step of advancing the funnel catheter with the radially self-expanding distal tip expanded distally; and
   maintaining a position of the funnel catheter with the radially self-expanding distal tip where the outer diameter of the radially self-expanding distal tip matches an inner diameter of a vessel being traversed or has reached a lower end of a vessel size range where the funnel tip can conform when advanced through a vessel with a smaller diameter than the tip.
4. The method of aspect 2, further comprising the step of generating a suction by proximally retracting the radially expanded innermost aspiration catheter with the clot ingested through a smaller inner diameter of the funnel catheter to aspirate any remaining clot fragments.
5. The method of aspect 1, further comprising the step of aspirating with one or more of the plurality of outer aspiration catheters to remove any remaining clot fragments.

## Claims

1. A catheter comprising:
a longitudinal axis;
a proximal elongate shaft comprising a proximal end, a distal end, a lumen, an inner liner, and a first plurality of wire braided segments comprising circumferential rings of cells; and
a distal tip section connected to the distal end of the proximal elongate shaft, the distal tip section comprising a proximal tubular body, a distal expansile section comprising a delivery configuration and an expanded clot capture configuration, and a second plurality of wire braided segments comprising a circumferential rings of cells;
the distal end of the distal expansile section comprising distal hoops where wires of the second plurality of wire braided segments invert to loop back through the second plurality of wire braided segments; and
the distal expansile section further comprising a larger expanded inner diameter when impinged radially by an ingested clot in the expanded clot capture configuration and a smaller delivery inner diameter in the delivery configuration.

2. The catheter of claim 1, at least a portion of the first plurality of wire braided segments comprising wires with a first material composition and at least a portion of the second plurality of wire braided segments comprising wires with a second material composition different than the first material composition.

3. The catheter of claim 1, a connection between the proximal elongate shaft and tip section comprising a proximal marker band bonded to the distal end of the proximal elongate shaft and the proximal end of the tip section.

4. The catheter of claim 3, at least a portion of the first plurality of wire braided segments and at least a portion of the second plurality of wire braided segments formed as a monolithic braid.

5. The catheter of claim 1, each cell of the first plurality of wire braided segments comprising a first braided segment braid angle.

6. The catheter of claim 1, the second plurality of wire braided segments comprises at least a first braid angle and a final braid angle, the final braid angle being less than the first braid angle.

7. The catheter of claim 5, distalmost cells of the second plurality of wire braided segments comprising a final braid angle in a range between approximately 40 degrees to approximately 120 degrees.

8. The catheter of claim 5, the distalmost cells of the second plurality of wire braided segments comprising a final braid angle in a range between approximately 85 degrees to approximately 95 degrees.

9. The catheter of claim 1, further comprising an axial spine extending distally from the proximal end of the proximal elongate shaft and inverting at a loop point through an opening in a cell of the second plurality of wire braided segments.

10. The catheter of claim 9, the one or more polymer tip jackets comprising a distalmost tip jacket having a hardness in a range between approximately 20 Shore A to approximately 72 Shore A.

11. The catheter of claim 1, further comprising an axial length between the distal end of the inner liner and the distal end of the distal tip section, the axial length being between approximately 1 mm and approximately 10 mm.

12. The catheter of claim 1, the distal tip section further comprising a longitudinal length in a range between approximately 100 mm to approximately 400 mm.

13. The catheter of claim 9, a spine loop point being axially located approximate the distal end of the inner liner.

14. The catheter of claim 9, the axial spine being interwoven with cells of the first plurality of wire braided segments and cells of the second plurality of wire braided segments.

15. The catheter of claim 2, at least a portion of the second plurality of wire braided segments comprising Nitinol wires.
